# EUROPEAN PATENT APPLICATION

(11) **EP 4 729 084 A1**
(43) Date of publication of application: **22.04.2026**
(21) Application number: 24749020.4
(22) Date of filing: 14.06.2024
(51) Int. Cl.: A61M 1/36, A61M 60/117, A61M 60/274, A61M 60/894

(54) **BLOOD GUIDE VALVE AND VENTRICULAR ASSIST PUMP DEVICE COMPRISING SAME**

(30) Priority: 16.06.2023 CN 202310716898
(71) Applicant: PulseCath B.V., 3621 ZA Breukelen (NL)
(72) Inventor: MALCHIN, Oren, 3621 ZA Breukelen (NL)
(74) Representative: V.O.
(86) International application number: PCT/IB2024/055842
(87) International publication number: WO 2024/257038

(57) **Abstract**

A blood flow guide valve and its ventricular auxiliary pump device, the blood flow guide valve includes: a valve seat, a valve body; wherein, the valve body is arranged in the inner chamber of the valve seat, and can rotate around the pivot in the first position, the second Rotate between positions; wherein, in the first position, the valve body closes the side hole of the valve seat and allows the blood flow in the valve seat to flow axially; in the second position, the valve body opens the the side hole connects the inside and outside of the valve seat, and closes the axial channel of the inner chamber of the valve seat, preventing the axial flow of blood flow in the valve seat on one side of the valve body; the pivot and the axis of the valve seat Different planes are vertical; the plane passing through the pivot and parallel to the valve seat axis divides the valve body into a first part and a larger second part, so that the blood flow guiding valve can open and close faster .

## Description

### TECHNICAL FIELD

The invention relates to a blood flow guide valve and a ventricle auxiliary pump device thereof, which are used for auxiliary pumping of blood in a blood circulation system.

### BACKGROUND OF THE INVENTION

Ventricular assist pumps of the prior art include a substantially rigid housing defining an interior space divided into two compartments by a flexible membrane. In operation, one compartment contains blood and the other contains gas. The ventricular assist pump is also connected to the heart by a single-lumen catheter having an inlet channel at its distal end and an outlet channel spaced therefrom for conveying blood through the catheter from the inlet to the exit. The ventricular assist pump has an opening for connection to a catheter in communication with the drive unit. The drive unit generates underpressure and overpressure (relative to the current pressures in the lumen and blood compartment) to alternately remove gas from and press gas into the gas chamber, respectively. During the suction phase, negative pressure pulls the membrane towards the first side of the housing, drawing blood through the inlet channel and, to some extent, also drawing blood into the membrane pump through the outlet channel of the single lumen catheter. This is followed by an ejection phase, when pressure pushes the membrane from a first side of the housing back to a second side of the housing, opposite the first side, thereby forcing blood out of the housing and into the lumen of the catheter. As blood is being expelled, very little blood flows out through the inlet channel , thereby alternately sucking and ejecting blood in a sequential pattern, resulting in a net pumping effect. The realization of the above-mentioned pumping effect is achieved through the cooperation of the blood flow guide valve to guide the blood flow.

However, the opening and closing response and sealing effect of the existing blood flow guiding valve still need to be improved, so as to further increase the response speed of the follow-up opening and closing, and the sealing effect of the valve body, so as to further improve and optimize the pumping capacity of the ventricular assist pump.

### BRIEF SUMMARY OF THE INVENTION

In order to overcome the above problems , the present invention provides a blood flow guiding valve , including: a valve seat and a valve body; wherein, the valve body is arranged in the inner cavity of the valve seat, and can be rotated around the pivot in the first position and the second position. In the first position, the valve body closes the side hole of the valve seat and allows the blood flow in the valve seat to flow axially; in the second position, the valve body opens the side hole. The hole connects the inner and outer fluids of the valve seat, and closes the axial channel of the inner chamber of the valve seat, preventing the axial flow of blood in the valve seat on one side of the valve body; the pivot axis is different from the axis of the valve seat, the surface is vertical; the valve body is an arc-shaped sheet structure, and its side wall is close to the corresponding side wall of the valve seat in the first position of closing the side hole, and the two are basically parallel; in the second position, the generatrix of the valve body is basically perpendicular to the axis of the valve seat; the plane passing through the pivot and parallel to the axis of the valve seat divides the valve body into the first part and the second part, wherein the first and the second parts longest busbar ratio is 0.75:1~0.85:1 for faster opening and closing response ; the oblique angle of the first part is larger than that of the second part.

As in the aforementioned blood flow guide valve, the valve body includes shaft holes at both ends; the outer contours between the shaft holes and the first part and the second part are connected by arc-shaped connecting parts; the valve outer edge of the first part of the body includes a first chamfered portion ; the first chamfered portion has a first chamfered chamfered surface ; the outer edge of the second portion of the valve body includes a second chamfered portion; the second chamfered portion has a second chamfered chamfered surface and an end peripheral chamfer.

In the aforementioned blood flow guiding valve , the outer edges of the first part and the second part have different wall thicknesses.

In the aforementioned blood flow guide valve , wherein the valve seat has an enlarged diameter section with an enlarged inner diameter in the lumen of the pipe section with side holes, and the inner lumens of the distal end and the proximal end of both ends are connected by a diameter reducing section.

In the aforementioned blood flow guiding valve , the distal end and the proximal end of the valve seat are used to engage with the catheter, so that the outer diameter of the valve seat is substantially consistent with the outer diameter of the catheter.

As mentioned above , the blood flow guide valve , wherein both the distal end and the proximal end have catheter insertion grooves for joining the catheter to form a firm connection and a smooth transition.

As mentioned above , the blood flow guide valve is made of titanium alloy or stainless steel.

A blood flow guide valve is also provided , including: a valve seat and a valve body; wherein, the valve body is arranged in the inner chamber of the valve seat and can rotate around a pivot between a first position and a second position; wherein, in the first position, the valve body closes the side hole of the valve seat and allows the blood flow in the valve seat to flow axially; in the second position, the valve body opens the side hole and allows the valve to the inside and outside of the seat are in fluid communication, and the axial channel of the inner cavity of the valve seat is closed to prevent the axial flow of blood flow in the valve seat on one side of the valve body; the pivot shaft is perpendicular to the axis of the valve seat; the valve body is an arc-shaped sheet structure, and at the first position of closing the side hole, its side wall is close to the corresponding side wall of the valve seat, and the two are basically parallel; at the second position, the busbar of the valve body is basically perpendicular to the axis of the valve seat, preferably 80 to 90 degrees; 85 degrees; the plane passing through the pivot and parallel to the axis of the valve seat divides the valve body into a first part and a second part, wherein the first , the longest busbar ratio of the two parts is 0.75:1~0.85:1 for faster opening and closing response ; the pivot is set away from the side hole and deviates from the main axis by about 43%~ 46% of the diameter .

As in the aforementioned blood flow guide valve , the outer edge of the first part of the valve body can be further divided into a first arc part, a second arc part, and a third arc part; the outer edge of the second part of the valve body, it can also be divided into the third arc part, the fourth arc part, and the fifth arc part; the area ratio of the first and second parts is 0.7:1~0.9:1.

As mentioned above, the blood flow guide valve, wherein each arc part is chamfered to reduce the turbulent flow caused by the valve body and reduce the risk of hemolysis and coagulation.

As mentioned above , the blood flow guide valve is made of titanium alloy or stainless steel.

As in the aforementioned blood flow guiding valve , the pivot is a movable pivot , and the corresponding inner wall of the valve seat has an axial pivot groove, so that the blood flow guiding valve rotates along the valve the pressure direction inside and outside the seat moves radially , so as to better block the side hole .

Also provided is a ventricular assist pump device comprising the aforementioned blood flow guiding valve , and further comprising a catheter and a diaphragm pump.

As in the aforementioned ventricular assist pump device, the catheter is a nickel-titanium reinforced single-channel catheter.

### Advantageous Effects

By optimizing the configuration and inclination of the valve body, the valve body can respond to opening and closing faster, reducing blood flow loss caused by response time delay.

Through the chamfering process, the joint surface between the valve body and the side hole of the valve seat is enlarged, and the side hole is better closed.

The edge of the valve body is chamfered to reduce the risk of blood coagulation caused by sharp edges.

The valve seat is provided with an enlarged diameter section, so that the valve body has a larger pivot arm, thereby having a larger busbar size, so that the valve body with a larger size can close the side hole.

### BRIEF DESCRIPTION OF THE DRAWINGS

Figure 1-2 is a schematic diagram of the use state of the ventricular assist device.
Figure 3 is a schematic diagram of the blood flow guide valve in the first and second positions respectively.
Figure 4 is a longitudinal sectional view of the blood flow guiding valves respectively in the first position.
Figure 5 is a schematic perspective view of a blood flow guide valve.

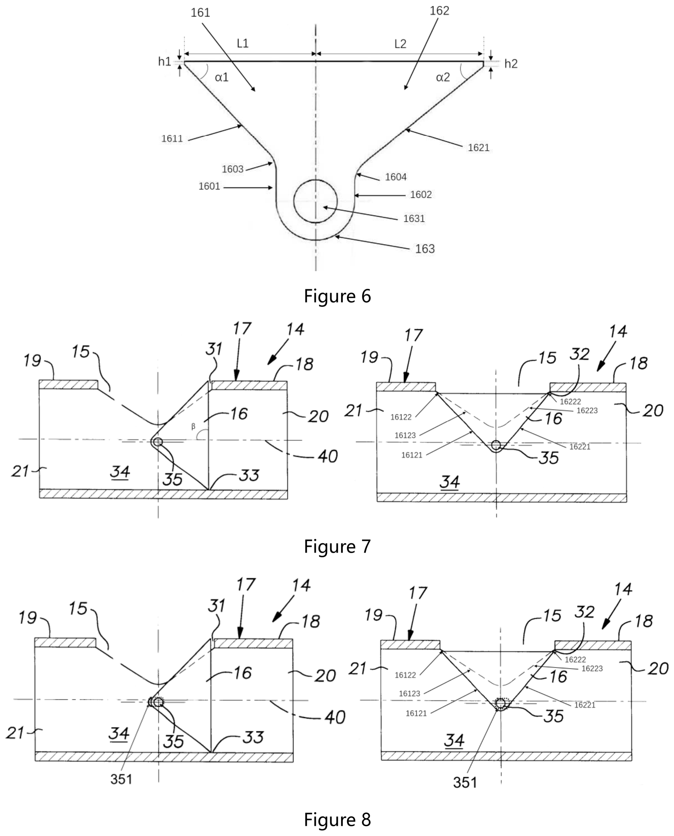

Figure 6 is a front view of the blood flow guiding valve.
Figure 7 is a longitudinal sectional view of the blood flow guiding valve in another embodiment when it is in the first and second positions;
Figure 8 is a longitudinal sectional view of the blood flow guiding valve in still another embodiment when it is in the first and second positions.

### EXAMPLES

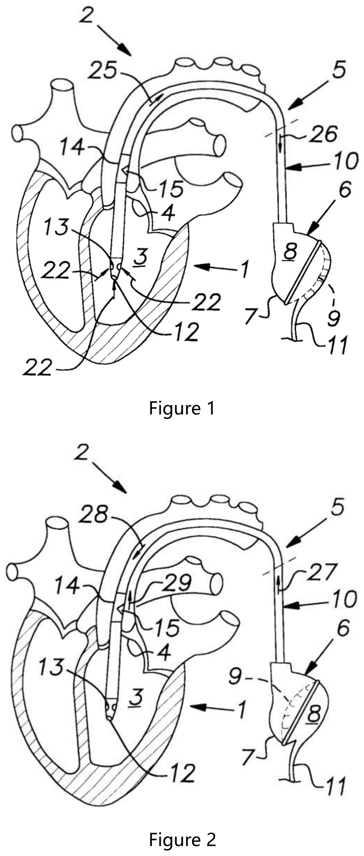

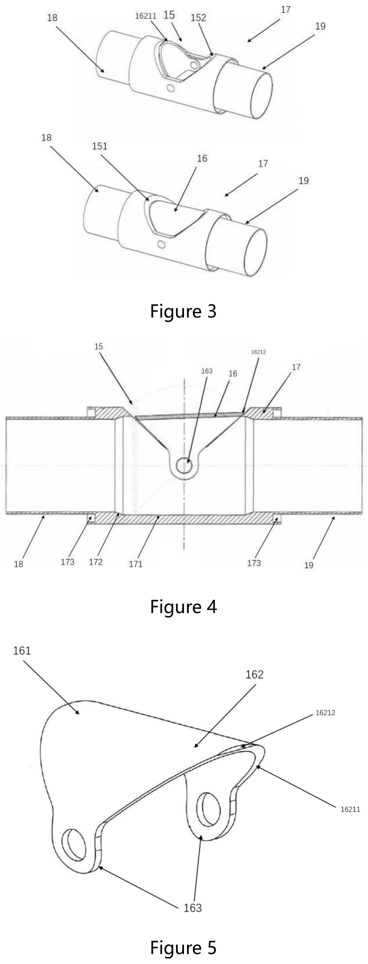

As shown in FIGS. 1-2 , the ventricular assist device 5 has a diaphragm pump 6 , a catheter 10 , a pneumatic catheter 11 , and a blood flow guiding valve 14 . As shown in FIG. 3 , the blood flow guide valve 14 includes: a valve seat 17 and a valve body 16; wherein, the valve body 16 is arranged in the inner cavity 34 of the valve seat, and can rotate around the pivot 35 of the valve seat 17 in the first position, the second Rotate between two positions; wherein, in the first position, the valve body 16 closes the side hole 15 of the valve seat 17, and allows the blood flow in the valve seat 17 to flow axially; in the second position, the valve body 16 opens the side hole 15 and The inner and outer fluids of the valve seat 17 are communicated, and the axial passage of the inner cavity 34 of the valve seat is closed to prevent the axial flow of blood flow in the valve seat 17 on one side of the valve body 16; the pivot 35 is perpendicular to the axis 40 of the valve seat .

Referring to accompanying drawings 5-6, the valve body 16 is an arc-shaped sheet structure made of titanium alloy or stainless steel. In the first position of the closed side hole 15, its side wall is close to the corresponding side wall of the valve seat 17, and the two are substantially parallel; in the second position, the generatrix of the valve body 16 is substantially perpendicular to the valve seat axis 40; through the pivot 35 and The plane parallel to the valve seat axis 40 divides the valve body 16 into a first part 161 and a second part 162; wherein, the ratio of the longest bus lines L1 and L2 of the first and second parts is 0.75:1~0.85:1, so as to be more Fast opening and closing response; the oblique angle α1 of the first portion 161 is greater than the oblique angle α2 of the second portion 162 .

The valve body 16 includes shaft holes 163 at both ends; the outer contours between the shaft holes 163 and the first part 161 and the second part 162 are respectively connected by arc-shaped connecting parts 1603 and 1604; the outer sides of the first part 161 of the valve body 16 The edge includes a first chamfered portion 1611; the first chamfered portion 1611 has a first chamfered chamfered surface; the outer edge of the second portion 162 of the valve body 16 includes a second chamfered portion 1621; the second chamfered portion 1621 It also has a second chamfered chamfered surface 16211 and an outer peripheral chamfer 16212 at the end. The chamfer 16212 at the outer periphery of the end is used to reduce the protrusion of the valve body relative to the valve seat 17 in the second position, reduce turbulent flow, and smooth the edge to prevent the end from being too sharp and easy to coagulate.

The arc connecting portions 1603 and 1604 have the same curvature; there are straight sections 1601 and 1602 between the arc connecting portions 1603 and 1604 and the shaft hole 163 , wherein the straight section 1601 is longer than the straight section 1602 .

The axial proximal end of the side hole 15 has a proximal oblique cut 152 to match the second chamfered chamfered surface 16211 to increase the contact area between the side wall of the valve body and the side wall of the valve seat, thereby increasing the closed area , Reduce assembly and machining accuracy.

In addition, the end edge of the second portion 162 has a wall thickness h2 that is greater than the greater wall thickness h1 of the end edge of the first portion 161, so that the second portion 162 has a wider second chamfer chamfer 16211 to The proximal beveled cutout 152 of the side hole 15 is engaged.

In a preferred embodiment, the axial distal end of the side hole 15 is symmetrically provided with the oblique cutout 152 at the proximal end, thereby reducing the requirement on the installation direction of the valve seat 17 during assembly.

The valve seat 17 has an enlarged diameter section 171 with an enlarged inner diameter in the lumen of the pipe section having the side hole 15 , which connects the lumens of the distal end 18 and the proximal end 19 at both ends through a diameter reducing section 172 . The enlarged diameter section 171 allows the valve body 16 to have a larger arm length/rotation radius when it is rotatable, thereby reducing the radial clearance between the valve body 16 and the valve seat 17 in the first position, and better aligning with the valve seat 17 The tube wall joint is closed. In addition, the diameter-expanding section 171 allows the valve body 16 to overlap on the axially proximal end of the side hole 15 with the proximal oblique cutout 152, so that the inner edge of the valve body 16 does not substantially exceed the inside of the distal portion 18 and the proximal portion 19. surface, to avoid influence on the diameter of the valve body and the blood flow; preferably, the inner surface of the valve is substantially consistent with the surface of the catheter.

The distal end 18 and the proximal end 19 of the valve seat 17 are used to engage the catheter 10, so that the outer diameter of the valve seat 17 is substantially consistent with the outer diameter of the catheter. In some embodiments, both the distal portion 18 and the proximal portion 19 have a catheter insertion groove 173 for engaging the catheter 10 to form a firm connection and a smooth transition.

In some embodiments, the angle β between the generatrix of the valve body 16 and the valve seat axis 40 is 80-90 degrees, preferably 85 degrees; in the state of the inclination angle B, when the blood flow is reversed, the valve body 16 can be more easily Rotate from the second position to the first position. In addition, in order to increase the pressure difference between the second part 162 and the first part 161 of the blood flow, the pivot 35 is set away from the side hole 15 and deviates from the main axis by about 43%~46% of the diameter, so that on the valve body 16 A greater torque is created, so that the lateral opening 15 is closed. For similar reasons, when the blood flow is reversed at the second position, the side hole 15 is opened by turning from the first position to the second position.

The outer edge of the first part 161 of the valve body 16 can be divided into a first arc part 16121, a second arc part 16122 and a third arc part 16123; the outer edge of the second part 162 of the valve body 16 can be divided into a third and fourth The arc part 16221, the fourth and fifth arc parts 16222, and the fifth and sixth arc parts 16223; the setting of the above arc parts can better fit the inner wall of the valve seat and the edge of the side hole to form a better seal. Wherein, each arc part is chamfered to reduce the turbulent flow caused by the valve body 16 and reduce the risk of hemolysis and coagulation. In addition, the area ratio of the first and second parts is 0.7:1~0.9:1.

In summary, the above-mentioned settings of the blood flow guiding valve enable the valve body 16 to quickly respond to blood flow direction and pressure changes, open and close the side hole 15 quickly, reduce leakage loss during blood pumping, and improve blood pumping capacity.

In the above embodiments, the pivot 35 and the shaft hole 163 can be respectively disposed on the valve seat 17 and the valve body 16 . In another embodiment, when the pivot 35 is arranged on the valve body 16, it is a movable pivot 35, and the inner wall of the corresponding valve seat 17 has an axial pivot groove 351, so that the blood flow guides the valve 14 can be moved while rotating, so as to better block the side hole 15 or the axial passage of the catheter. For example, when the valve body 16 is in the second position, the valve body 16 moves to the distal end under the action of the pressure inside the catheter, thereby reducing the gap 31 , thereby reducing the return of blood from outside the catheter into the catheter lumen.

In summary, by optimizing the configuration and inclination of the valve body, the blood flow guiding valve and its ventricular assist pump device enable the valve body to respond to opening and closing faster and reduce blood flow loss caused by response time delay; treatment, so that the joint surface of the valve body and the side hole of the valve seat is enlarged, and the side hole is better closed; the edge of the valve body is chamfered to reduce the risk of coagulation caused by the sharp edge; The valve body has a larger rotating arm, so it has a larger busbar size, and the side hole is closed with a larger valve body, which further improves the response speed of the follow-up opening and closing, and the sealing effect of the valve body, so as to further improve and optimize The pumping capacity of the ventricular assist pump.

Other embodiments of the present disclosure will be readily apparent to those skilled in the art from consideration of the specification and practice of the invention disclosed herein. This application is intended to cover any modification, use or adaptation of the present disclosure, and these modifications, uses or adaptations follow the general principles of the present disclosure and include common knowledge or conventional technical means in the technical field not disclosed in the present disclosure . The specification and examples are to be considered exemplary only, with a true scope and spirit of the disclosure being indicated by the following claims.

It should be understood that the present disclosure is not limited to the precise constructions which have been described above and shown in the drawings, and various modifications and changes may be made without departing from the scope thereof. The scope of the present disclosure is limited only by the appended claims.

## Claims

1. A blood flow guide valve , comprising: a valve seat and a valve body; wherein, the valve body is arranged in the inner cavity of the valve seat and can rotate around a pivot between the first position and the second position; its characteristic is :
wherein, in the said first position, the valve body closes the side hole of the valve seat and allows the blood flow in the valve seat to flow axially;
in the second position, the valve body opens the side hole and makes the inside and outside of the valve seat communicate with each other, and closes the axial channel of the inner chamber of the valve seat, preventing the inside of the valve seat on one side of the valve body from Axial blood flow;
the pivot is perpendicular to the axis of the valve seat;
the valve body is an arc-shaped sheet structure, and its side wall is close to the corresponding side wall of the valve seat in the first position of closing the side hole, and the two are basically parallel; in the second position, the valve body The bus bar is basically perpendicular to the axis of the valve seat;
the valve body is divided into a first part and a second part by a plane passing through the pivot and parallel to the valve seat axis, wherein the longest busbar ratio of the first and second parts is 0.75:1~0.85:1, for faster opening and closing response .

2. The blood flow guiding valve according to claim 1 , wherein the bevel angle of the first portion is larger than the bevel angle of the second portion.

3. The blood flow guiding valve according to claim 2 , the valve body further includes shaft holes at both ends; the outer contours between the shaft holes and the first part and the second part are connected by arc-shaped connecting parts;
the outer edge of the first part of the valve body includes a first chamfered portion; the first chamfered portion has a first chamfered chamfered surface;
the outer edge of the second part of the valve body includes a second chamfered portion; the second chamfered portion has a second chamfered chamfered surface and an end peripheral chamfer.

4. The blood flow guiding valve according to any one of claims 1-3, wherein the outer edges of the first part and the second part have different wall thicknesses.

5. The blood flow guiding valve according to claim 1 , wherein the valve seat has an enlarged diameter section with an enlarged inner diameter in the inner cavity of the pipe section with a side hole, and the distal end and the proximal end of both ends are connected by a diameter reducing section of the inner cavity.

6. The blood flow directing valve as claimed in claim 1 , wherein the distal end and the proximal end of the valve seat are adapted to engage the catheter such that the outer diameter of the valve seat coincides with the outer diameter of the catheter.

7. The blood flow guiding valve according to claim 6 , wherein both the distal end and the proximal end have catheter insertion grooves for joining the catheter to form a firm connection and a smooth transition.

8. The blood flow guiding valve according to claim 1, in the second position, the busbar of the valve body is basically 80-90 degrees to the valve seat axis, preferably 85 degrees; the pivot is set far away from the side, the hole deviates from the main axis at about 43%~46% of the diameter.

9. The blood flow guide valve according to claim 8 , the outer edge of the first part of the valve body can be divided into a first arc part, a second arc part and a third arc part; The outer edge of the second part of the valve body can be further divided into a third arc part, a fourth arc part, and a fifth arc part; the area ratio of the first part and the second part is 0.7:1~0.9:1.

10. The blood flow guiding valve according to claim 9 , wherein each arc portion is chamfered to reduce the turbulent flow caused by the valve body and reduce the risk of thrombus caused by hemolysis and coagulation.

11. The blood flow guide valve according to any one of claims 8-10, wherein the pivot is a movable pivot , and the corresponding inner wall of the valve seat has an axial pivot groove, so that the blood flow When the guide valve rotates, it moves radially along the inner and outer pressure direction of the valve seat , so as to better block the side hole .

12. The blood flow guiding valve according to any one of claims 1-11 , which is made of titanium alloy or stainless steel .

13. A ventricular assist pump device comprising the blood flow guiding valve according to any one of claims 1 to 12, **characterized in that** it also comprises a catheter and a diaphragm pump.

14. The ventricular assist pump device of claim 13, said catheter being a nickel titanium reinforced single channel catheter.
